(19) Europäisches Patentamt
European Patent Office
Office européen des brevets

(11) EP 1 499 589 B1

(12) **FASCICULE DE BREVET EUROPEEN**

(45) Date de publication et mention de la délivrance du brevet:
**02.05.2007 Bulletin 2007/18**

(21) Numéro de dépôt: **03740634.5**

(22) Date de dépôt: **17.04.2003**

(51) Int Cl.:
***C07D 211/26*** *(2006.01)* ***A61K 31/445*** *(2006.01)*
***A61P 25/00*** *(2006.01)*

(86) Numéro de dépôt international:
**PCT/FR2003/001232**

(87) Numéro de publication internationale:
**WO 2003/089411 (30.10.2003 Gazette 2003/44)**

(54) **DERIVES DE N-¬PHENYL(PIPERIDIN-2-YL)METHYL|BENZAMIDE, LEUR PREPARATION ET LEUR APPLICATION EN THERAPEUTIQUE**

N-¬PHENYL(PIPERIDIN-2-YL)METHYL|BENZAMIDDERIVATE,VERFAHREN ZU IHRER HERSTELLUNG UND IHRE THERAPEUTISCHE ANWENDUNG

DERIVATIVES OF N-PHENYL(PIPERIDIN-2-YL)METHYL BENZAMIDE, THE PREPARATION METHOD THEREOF AND APPLICATION OF SAME IN THERAPEUTICS

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **19.04.2002 FR 0204916**

(43) Date de publication de la demande:
**26.01.2005 Bulletin 2005/04**

(73) Titulaire: **Sanofi-Aventis**
**75013 Paris (FR)**

(72) Inventeurs:
- **DARGAZANLI, Gihad**
  **F-94230 Cachan (FR)**
- **ESTENNE-BOUHTOU, Geneviève**
  **F-94550 Chevilly-Larue (FR)**
- **MAGAT, Pascale**
  **F-94380 Chilly-Mazarin (FR)**
- **MARABOUT, Benoit**
  **F-91300 Massy (FR)**
- **MEDAISKO, Florence**
  **F-94100 Saint Maur des Fosses (FR)**
- **ROGER, Pierre**
  **F-78180 Montigny-le-Bretonneux (FR)**
- **SEVRIN, Mireille**
  **F-75014 Paris (FR)**
- **VERONIQUE, Corinne**
  **F-92160 Antony (FR)**

(74) Mandataire: **Ludwig, Jacques et al**
**Sanofi-Aventis**
**174 avenue de France**
**75013 Paris (FR)**

(56) Documents cités:
**EP-A- 0 499 995**
**WO-A-01/81308**
**WO-A-99/45011**
**US-A- 5 254 569**

EP 1 499 589 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

Les composés de l'invention répondent à la formule générale (I)

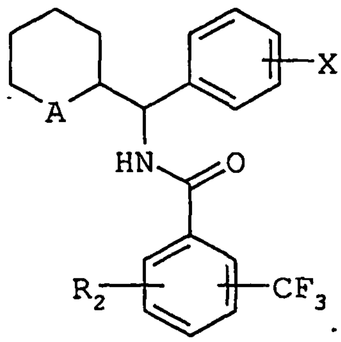

(I)

dans laquelle A représente
soit un groupe de formule générale N-$R_1$ dans laquelle $R_1$ représente soit un atome d'hydrogène, soit un groupe ($C_1$-$C_7$) alkyle linéaire ou ramifié éventuellement substitué par un ou plusieurs atomes de fluor, soit un groupe ($C_4$-$C_7$)cycloalkyle, soit un groupe ($C_3$-$C_7$)cycloalkyl-($C_1$-$C_3$)alkyle, soit un groupe phényl($C_1$-$C_3$)alkyle éventuellement substitué par un ou deux groupes hydroxy ou méthoxy, soit un groupe ($C_2$-$C_4$)alcényle, soit un groupe ($C_2$-$C_4$)alcynyle,
soit un groupe de formule générale $N^+(O^-)R_1$ dans laquelle $R_1$ est tel que défini ci-dessus,
soit encore un groupe de formule générale $N^+(R')R_1$ dans laquelle R' représente un groupe ($C_1$-$C_7$)alkyle linéaire ou ramifié et $R_1$ est tel que défini ci-dessus,
X représente un atome d'hydrogène ou un ou plusieurs substituants choisis parmi les atomes d'halogènes et les groupes trifluorométhyle, ($C_1$-$C_4$)alkyle linéaire ou ramifié et ($C_1$-$C_4$)alcoxy,
$R_2$ représente soit un atome d'hydrogène, soit un ou plusieurs substituants choisis parmi les atomes d'halogènes et les groupes trifluorométhyle, ($C_1$-$C_4$)alkyles, ($C_1$-$C_4$)alcoxy, amino de formule générale $NR_3R_4$ dans laquelle $R_3$ et $R_4$ représentent chacun, indépendamment l'un de l'autre, un atome d'hydrogène ou un groupe ($C_1$-$C_4$)alkyle, ou forment avec l'atome d'azote qui les porte un cycle pyrrolidine, pipéridine ou morpholine, soit un groupe phényle éventuellement substitué par un atome ou un groupe tels que définis pour le symbole X ci-dessus.

Les composés de formule générale (I) peuvent exister sous forme du racémate thréo (1*R*, 2*R* ; 1*S*, 2*S*) ou sous forme d'énantiomères (1*R*, 2*R*) ou (1*S*,2*S*) ; ils peuvent exister à l'état de bases libres ou de sels d'addition à des acides.

Des composés de structure analogue à celle des composés de l'invention sont décrits dans le brevet US-5254569 comme analgésiques, diurétiques, anticonvulsivants, anesthésiques, sédatifs, cérébroprotecteurs, par un, mécanisme d'action sur les récepteurs opiacés. D'autres composés de structure analogue sont décrits dans la demande de brevet EP-0499995 comme antagonistes 5-$HT_3$ utiles dans le traitement des désordres psychotiques, des maladies neurologiques, des symptômes gastriques, des nausées et des vomissements. Le document WO 01/81308 décrit, lui, des composés inhibiteurs du transport de glycine qui sont utiles dans le traitement de désordres du système nerveux central.

Les composés de l'invention présentent une activité particulière comme inhibiteurs spécifiques des transporteurs de la glycine glyt1 et/ou glyt2.

Les composés préférés comme inhibiteurs du transporteur glyt1 sont de configuration (1*S*,2*S*) avec $R_2$ représentant un ou plusieurs atomes d'halogènes ou groupes trifluorométhyle, alors que les composés préférés comme inhibiteurs du transporteur glyt2 sont de configuration (1*R*,2*R*) avec $R_2$ représentant un atome d'halogène et un groupe amino de formule générale $NR_3R_4$.

Les composés de formule générale (I) dans laquelle A représente un groupe de formule générale N-R1, où $R_1$ est différent d'un atome d'hydrogène peuvent être préparés par un procédé illustré par le schéma 1 qui suit.

Schéma 1

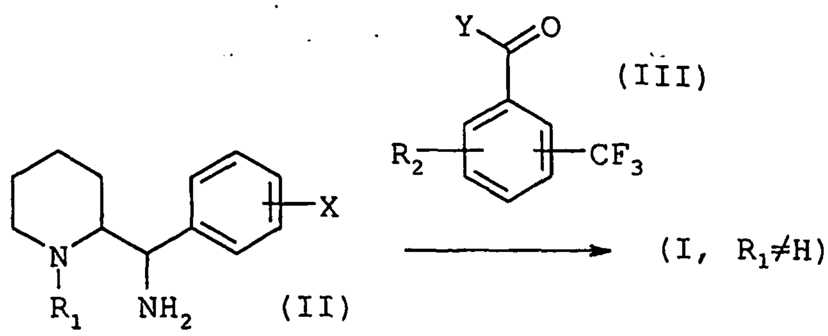

On effectue un couplage d'une diamine de formule générale (II), dans laquelle $R_1$ et X sont tels que définis ci-dessus (avec $R_1$ différent d'un atome d'hydrogène) avec un acide activé ou un chlorure d'acide de formule générale (III) dans laquelle Y représente un groupe nucléofuge, tel qu'un un atome d'halogène et $R_2$ est tel que défini ci-dessus, en utilisant les méthodes connues de l'homme du métier.

La diamine de formule générale (II) peut être préparée par un procédé illustré par le schéma 2 qui suit.

Schéma 2

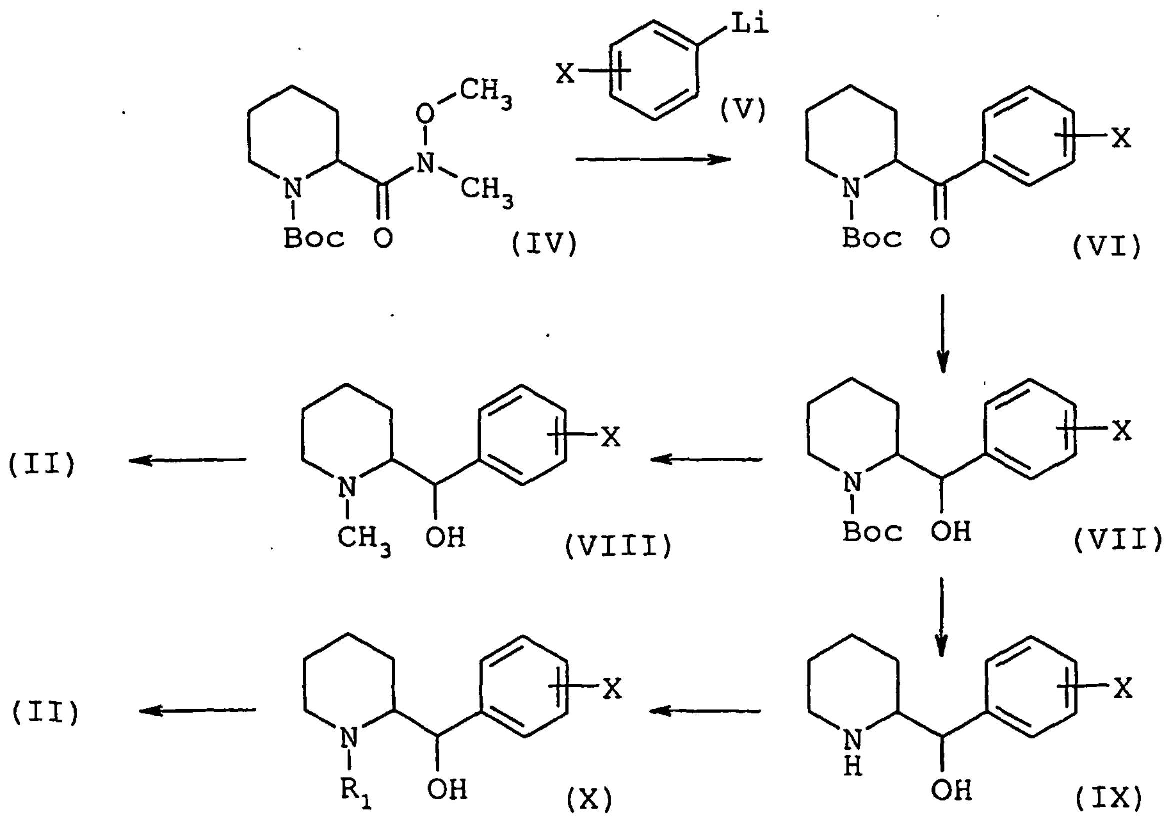

On fait réagir l'amide de Weinreb de formule (IV) avec le dérivé de phényllithium de formule générale (V), dans laquelle X est tel que défini ci-dessus, dans un solvant éthéré tel que l'éther diéthylique, entre -30°C et la température ambiante ; on obtient une cétone de formule générale (VI)que l'on réduit en alcool de configuration thréo de formule générale (VII) par un agent réducteur tel que le K-Selectride® ou le L-Selectride® (tri-sec-butylborohydrure de potassium ou de lithium), dans un solvant éthéré tel que le tétrahydrofurane, entre -78°C et la température ambiante. Le carbamate

de formule générale (VII) peut ensuite être réduit en *N*-méthylaminoalcool thréo de formule générale (VIII) par action d'un hydrure mixte tel que l'hydrure double d'aluminium et de lithium, dans un solvant éthéré tel que le tétrahydrofurane, entre la température ambiante et la température de reflux. On transforme ensuite l'alcool thréo de formule générale (VIII) en intermédiaire thréo de formule générale (II) où $R_1$ représente un groupe méthyle en deux étapes : on transforme d'abord la fonction alcool en groupe nucléofuge, par exemple un groupe méthanesulfonate par action du chlorure de méthylsulfonyle, dans un solvant chloré tel que le dichlorométhane, et en présence d'une base telle que la triéthylamine, entre 0°C et la température ambiante, puis on fait réagir le groupe nucléofuge avec de l'ammoniac liquéfié à -50°C, dans un alcool tel que l'éthanol, dans un milieu clos tel qu'un autoclave, entre -50°C et la température ambiante.

On peut également déprotéger le carbamate de formule générale (VII) au moyen d'une base forte telle que la potasse aqueuse, dans un alcool tel que le méthanol pour obtenir l'aminoalcool thréo de formule générale (IX), procéder ensuite à une *N*-alkylation au moyen d'un dérivé halogéné de formule $R_1Z$, dans laquelle $R_1$ est tel que défini ci-dessus, mais différent d'un atome d'hydrogène, et Z représente un atome d'halogène, en présence d'une base telle que le carbonate de potassium, dans un solvant polaire tel que le *N,N*-diméthylformamide, entre la température ambiante et 100°C. On traite ensuite l'alcool de formule générale (X) ainsi obtenu comme décrit à propos de l'alcool de formule générale (VIII).

Une autre variante de procédé, illustrée par le schéma 3 qui suit, peut être utilisée dans le cas où $R_1$ représente un groupe méthyle et X représente un atome d'hydrogène.

On quaternarise la pyridineoxime de formule (XI), par exemple par action du trifluorométhanesulfonate de méthyle, dans un solvant éthéré tel que l'éther diéthylique à température ambiante. On soumet ensuite le sel de pyridinium ainsi obtenu, de formule (XII) à une hydrogénation sous atmosphère d'hydrogène, en présence d'un

Schéma 3

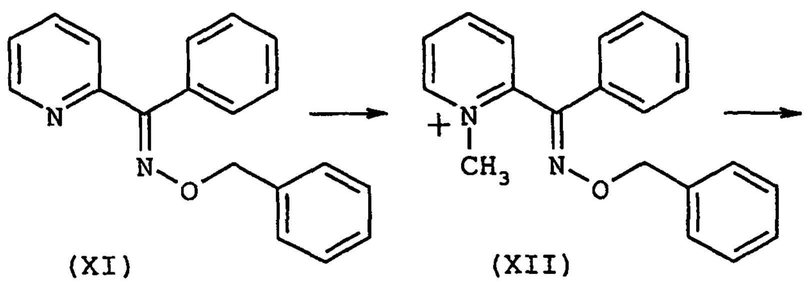

(II, $R_1$=$CH_3$, X=H)

(XI) (XII)

catalyseur tel que l'oxyde de platine, dans un mélange d'alcool et d'acide aqueux tel que l'éthanol et l'acide chlorhydrique 1N. On obtient la diamine de formule générale (II) où $R_1$ représente un groupe méthyle et X représente un atome d'hydrogène sous forme d'un mélange des deux diastéréoisomères thréo/érythro 9/1. On peut la salifier, par exemple avec de l'acide oxalique, puis purifier par recristallisation de l'oxalate formé dans un mélange d'alcool et d'un solvant éthéré tel que le méthanol et l'éther diéthylique, pour obtenir le diastéréoisomère thréo (1*R*, 2*R* ; 1*S*,2*S*) pur.

Les composés de formule générale (I) dans laquelle A représente un groupe de formule générale $NR_1$ où $R_1$ représente un atome d'hydrogène peuvent être préparés par un procédé illustré par le schéma 4 qui suit.

Schéma 4

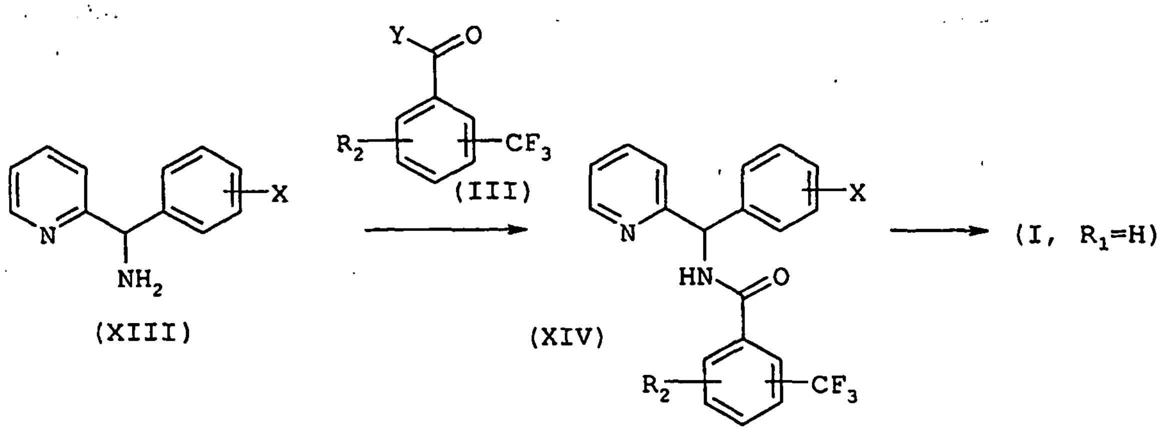

A partir de l'amine de formule générale (XIII), dans laquelle X est tel que défini ci-dessus, on effectue un couplage avec un acide activé ou un chlorure d'acide, tel que décrit ci-dessus, de formule générale (III), selon des méthodes connues de l'homme du métier, pour obtenir le composé de formule générale (XIV). Finalement on effectue une hydrogénation de ce dernier, par exemple par l'hydrogène en présence d'un catalyseur tel que le platine à 5% sur charbon, dans un solvant acide tel que l'acide acétique glacial, pour finalement obtenir un composé de formule générale (I) dans laquelle $R_1$ représente un atome d'hydrogène.

Une autre méthode consiste, suivant le schéma 2, à utiliser un composé de formule générale (I) dans laquelle $R_1$ représente soit un groupe phénylméthyle éventuellement substitué, et à déprotéger l'azote du cycle pipéridine, par exemple par un agent oxydant ou par un acide de Lewis tel que le tribromure de bore, ou par hydrogénolyse, soit un groupe alcényle, de préférence un groupe allyle, suivie d'une déprotection par un complexe de $Pd^0$, pour obtenir un composé de formule générale (I) dans laquelle $R_1$ représente un atome d'hydrogène.

Les composés de formule générale (I) dans laquelle A représente un groupe de formule générale $N^+(O^-)R_1$ peuvent être préparés à partir des composés de formule générale (I) dans laquelle A représente un groupe de formule générale $N\text{-}R_1$, dans laquelle $R_1$ est tel que décrit ci-dessus, par réaction avec un agent oxydant, par exemple l'acide 3-chloroperbenzoïque, dans un solvant chloré tel que le dichlorométhane, à une température comprise entre 0°C et l'ambiante.

Les composés de formule générale (I) dans laquelle A représente un groupe de formule générale $N^+(R')R_1$, peuvent être préparés à partir de composés de formule générale (I) dans laquelle A représente un groupe de formule générale $N\text{-}R_1$, par réaction avec un halogénure d'alkyle de formule générale R'-Z, dans laquelle R' est tel que défini ci-dessus et Z représente un atome d'halogène, dans un solvant polaire tel que l'acétonitrile, à une température comprise entre l'ambiante et 100°C.

Par ailleurs, les composés chiraux de formule générale (I) correspondant aux énantiomères (1*R*,2*R*) ou (1*S*, 2*S*) du diastéréoisomère thréo peuvent être également obtenus par séparation des composés racémiques par chromatographie liquide à haute performance (CLHP) sur colonne chirale, ou par dédoublement de l'amine racémique de formule générale (II) avec utilisation d'un acide chiral, tel que l'acide tartrique, l'acide camphorsulfonique, l'acide dibenzoyltartrique, la *N*-acétylleucine, par la recristallisation fractionnée et préférentielle d'un sel diastéréoisimérique dans un solvant de type alcool, soit par synthèse énantiosélective selon le schéma 2, avec utilisation d'un amide de Weinreb chiral de formule générale (IV).

L'amide de Weinreb de formule (IV) racémique ou chiral peut être préparé selon une méthode analogue à celle décrite dans *Eur. J. Med. Chem.,* **35,** (2000), 979-988 et *J. Med. Chem.*, **41**, (1998), 591-601. Le composé phényllithié de formule générale (V) où X représente un atome d'hydrogène est disponible dans le commerce. Ses dérivés substitués peuvent être préparés selon une méthode analogue à celle décrite dans *Tetra. Lett.*, **57**, 33, (1996), 5905-5908. La pyridylcétoxime éther de formule (XI) peut être préparée selon une méthode analogue à celle décrite dans la demande de brevet EP-0366006. L'amine de formule générale (IX) dans laquelle X représente un atome d'hydrogène peut être préparée en série chirale selon une méthode décrite dans le brevet US-2928835. Enfin, l'amine de formule générale (XIII) peut être préparée selon une méthode analogue à celle décrite dans *Chem. Pharm. Bull.*,**32, 12,** (1984), 4893-4906 et *Synthesis,* (1976), 593-595.

Les acides et chlorures d'acides de formule générale (III) sont disponibles dans le commerce, sauf dans le cas de l'acide 4-amino-3-chloro-5-trifluorométhylbenzoique. On peut préparer ce dernier par chloration de l'acide 4-amino-5-trifluorométhylbenzoïque avec le chlorure de sulfuryle dans un solvant chloré tel que le chloroforme, selon une méthode analogue à celle décrite dans *Arzneim. Forsch.,* **34,** 11a, (1984), 1668-1679.

Les exemples qui vont suivre illustrent la préparation de quelques composés de l'invention. Les microanalyses élémentaires, et les spectres I.R. et R.M.N. et la CLHP sur colonne chirale confirment les structures et les puretés énantiométriques des composés obtenus.

Les numéros indiqués entre parenthèses dans les titres des exemples correspondent à ceux de la 1ère colonne du tableau donné plus loin.

Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

<u>Exemple 1</u> (Composé N°33).

Chlorhydrate de thréo-2-chloro-*N*-[(1-éthylpipéridin-2-yl)phénylméthyl]-3-trifluorométhylbenzamide 1:1.

1.1. 2-benzoylpipéridine-1-carboxylate de 1,1-diméthyléthyle.

Dans un ballon de 250 ml, sous atmosphère d'argon, on introduit 8,0 g (29,4 mmoles) de 2-(*N*-méthoxy-*N*-méthylcarbamoyl)pipéridine-1-carboxylate de 1,1-diméthyléthyle dans 100 ml d'éther diéthylique anhydre, on refroidit le milieu à -25°C, on ajoute, goutte à goutte, 16 ml (29,4 mmoles) d'une solution 1,8M de phényllithium dans un mélange 70/30 de cyclohexane et d'éther diéthylique et on maintient l'agitation pendant 2 h.

Après hydrolyse avec une solution aqueuse saturée de chlorure de sodium on sépare la phase aqueuse, on l'extrait avec de l'acétate d'éthyle, on sèche la phase organique sur sulfate de sodium, on la filtre et on concentre le filtrat sous pression réduite et on purifie le résidu par chromatographie sur colonne de gel de silice en éluant avec un mélange d'acétate d'éthyle et de cyclohexane.

On obtient 2 g de solide blanc.

1.2. thréo-[hydroxy(phényl)méthyl]pipéridine-1-carboxylate de 1,1-diméthyléthyle.

Dans un ballon de 250 ml, sous atmosphère d'argon, on introduit 2,0 g (6,9 mmoles)) de 2-benzoylpipéridine-1-carboxylate de 1,1-diméthyléthyle dans 30 ml d'éther diéthylique anhydre, on refroidit la solution à -78°C, on ajoute, goutte à goutte, 20,7 ml (20,7 mmoles) d'une solution 1M de tri-sec-butylborohydrure de lithium dans l'éther diéthylique et on maintient l'agitation pendant 3 h.

On hydrolyse le mélange avec 16 ml d'eau et 16 ml d'une solution aqueuse à 35% de peroxyde d'hydrogène, et on laisse le mélange revenir à température ambiante en l'agitant pendant 2 h.

On le dilue avec de l'eau et de l'acétate d'éthyle, on sépare la phase aqueuse, et on l'extrait avec de l'acétate d'éthyle. Après lavage des phases organiques réunies, séchage sur sulfate de sodium et évaporation du solvant sous pression réduite, on purifie le résidu par chromatographie sur colonne de gel de silice en éluant avec un mélange d'acétate d'éthyle et de cyclohexane.

On obtient 2,0 g de produit huileux.

1.3. thréo-phényl(pipéridin-2-yl)méthanol.

Dans un ballon de 250 ml on place une solution de 2,0 g (6,9 mmoles) de thréo-[hydroxy(phényl)méthyl] pipéridine-1-carboxylate de 1,1-diméthyléthyle dans 40 ml de méthanol, on ajoute une solution aqueuse de potasse préparée à partir de 2 g de potasse en pastilles et 20 ml d'eau, et on chauffe le mélange au reflux pendant 2 h.

On refroidit le mélange, on évapore le solvant sous pression réduite, on ajoute de l'eau et on extrait le mélange plusieurs fois avec du dichlorométhane. après lavage des phases organiques réunies, séchage sur sulfate de magnésium, filtration et évaporation du solvant sous pression réduite on obtient 1 g de solide blanc.

Point de fusion : 172-174°C.

1.4. thréo-(1-éthylpipéridin-2-yl)phénylméthanol.

Dans un ballon de 100 ml on place une solution de 1 g (5,2 mmoles) de thréo-phényl(pipéridin-2-yl)méthanol dans 30 ml de *N,N*-diméthylformamide anhydre, on ajoute 0,39 ml (5,2 mmoles) de bromoéthane et 0,8 g (5,8 mmoles) de carbonate de potassium, et on chauffe le mélange à 80°C pendant 2 h.

On le refroidit à température ambiante, on l'hydrolyse par addition d'eau et on l'extrait plusieurs fois avec de l'acétate d'éthyle. Après lavage des phases organiques réunies avec de l'eau puis une solution aqueuse saturée de chlorure de sodium, séchage sur sulfate de magnésium, filtration et évaporation du solvant sous pression réduite, on purifie le résidu

par chromatographie sur colonne de gel de silice en éluant avec un mélange de dichlorométhane et de méthanol. On obtient 0,8 g de composé huileux.

1.5. thréo-(1-éthylpipéridin-2-yl)phénylméthanamine.

Dans un ballon de 100 ml, sous atmosphère d'argon, on introduit 0,8 g (3,65 mmoles) de thréo-(1-éthylpipéridin-2-yl)phénylméthanol et 0,48 ml (3,65 mmoles) de triéthylamine dans 20 ml de dichlorométhane anhydre, on refroidit le mélange à 0°C, on ajoute 0,28 ml (3,63 mmoles) de chlorure de méthanesulfonyle et on laisse le mélange revenir lentement à température ambiante pendant 2 h et on le concentre sous pression réduite.
Dans un autoclave muni d'une agitation magnétique et refroidi à -50°C on introduit de l'ammoniac liquéfié et on ajoute le méthanesulfonate préalablement préparé en solution dans 10 ml d'éthanol absolu, on ferme l'autoclave et on maintient l'agitation pendant 48 h.
On transvase le mélange dans un ballon, on le concentre sous pression réduite et on purifie le résidu par chromatographie sur colonne de gel de silice en éluant avec un mélange de dichlorométhane et de méthanol.
On obtient 0,3 g de composé huileux qu'on utilise tel quel dans l'étape suivante.

1.6. Chlorhydrate de thréo-2-chloro-*N*-[(1-éthylpipéridin-2-yl)phénylméthyl]-3-trifluorométhylbenzamide 1:1.

Dans un ballon de 50 ml 0,3 g (1,37 mmole) d'acide 2-chloro-3-trifluorométhylbenzoïque, 0,26 g (1,37 mmole) de chlorhydrate de 1-[3-(diméthylamino)propyl]-3-éthylcarbodiimide, 0,19 g (1,37 mmole) de 1-hydroxybenzotriazole en solution dans 10 ml de dichlorométhane et on agite le mélange à température ambiante pendant 30 min.
On ajoute 0,3 g (1,37 mmole) de thréo-(1-éthylpipéridin-2-yl)phénylméthanamine en solution dans quelques ml de dichlorométhane et on poursuit l'agitation pendant 5 h.
On hydrolyse le mélange avec de l'eau, on l'extrait plusieurs fois avec du dichlorométhane. Après lavage des phases organiques avec de l'eau puis avec une solution aqueuse de soude 1N, séchage sur sulfate de magnésium, filtration et évaporation du solvant sous pression réduite, on purifie le résidu par chromatographie sur colonne de gel de silice en éluant avec un mélange de dichlorométhane et de méthanol.
On obtient 0,25 g de produit huileux.
On dissout ce dernier dans quelques ml de propan-2-ol, on ajoute 5,9 ml d'une solution 0,1N d'acide chlorhydrique dans le propan-2-ol, et on concentre le mélange sous pression réduite afin de réduire le volume du solvant. Après trituration on isole finalement 0,15 g de chlorhydrate sous forme d'un solide blanc.
Point de fusion : 230-232°C.

<u>Exemple 2</u> (Composé N°18).

Chlorhydrate de 2-chloro-*N*-[(1*S*)-[(2*S*)-1-méthylpipéridin-2-yl]pliénylméthyl]-3-trifluorométhylbenzamide 1:1.

2.1. (2*S*)-2-benzoylpipéridine-1-carboxylate de 1,1-diméthyléthyle.

Dans un ballon de 500 ml, sous atmosphère d'azote, on introduit 11,8 g (43,3 mmoles) de (2*S*)-2-(*N*-méthoxy-*N*-méthylcarbamoyl)pipéridine-1-carboxylate de 1,1-diméthyléthyle dans 100 ml d'éther diéthylique anhydre, on refroidit le milieu à -23°C, on ajoute, goutte à goutte, 21,6 ml (43,2 mmoles) d'une solution 1,8M de phényllithium dans un mélange 70/30 de cyclohexane et d'éther diéthylique et on agite le mélange à température ambiante pendant 3 h. Après hydrolyse avec une solution aqueuse saturée de chlorure de sodium on sépare la phase aqueuse et on l'extrait avec de l'acétate d'éthyle. On sèche la phase organique sur sulfate de sodium, on la filtre, on la concentre sous pression réduite et on purifie le résidu par chromatographie sur colonne de gel de silice en éluant avec un mélange d'acétate d'éthyle et de cyclohexane.
On obtient 4,55 g de produit solide.
Point de fusion : 123-125°C.

$[\alpha]_D^{25}=-25,4°$ (c=2,22 ; $CH_2Cl_2$) ee=97,2%.

2.2. (1*S*)-2-[(2*S*)-hydroxy(phényl)méthyl]pipéridine-1-carboxylate de 1,1-diméthyléthyle.

Dans un ballon de 500 ml, sous atmosphère d'azote, on introduit 4,68 g (16,2 mmoles) de (2*S*)-2-benzoylpipéridine-1-carboxylate de 1,1-diméthyléthyle dans 170 ml de tétrahydrofurane anhydre, on refroidit la solution à -78°C, on ajoute, goutte à goutte , 48,5 ml (48,5 mmoles) d'une solution 1M de L-Selectride® (tri-*sec*-butylborohydrure de lithium) dans le tétrahydrofurane, et on agite le mélange à température ambiante pendant 5 h.

On l'hydrolyse à froid lentement avec 34 ml d'eau et 34 ml d'une solution aqueuse à 35% de peroxyde d'hydrogène, et on laisse le mélange revenir à température ambiante en l'agitant pendant 2 h.
On le dilue avec de l'eau et de l'acétate d'éthyle, on sépare la phase aqueuse, on l'extrait avec de l'acétate d'éthyle. Après lavage des phases organiques réunies, séchage, séchage sur sulfate de sodium, filtration et évaporation on purifie le résidu par chromatographie sur colonne de gel de silice en éluant avec un mélange d'acétate d'éthyle et de cyclohexane.
On obtient 4,49 g d'une huile jaune pâle.

$[\alpha]_D^{25}=+63,75°$ (c=0,8 ; $CH_2Cl_2$) ee=97,8 %.

2.3. (1*S*)-[(2*S*)-(1-méthylpipéridin-2-yl)]phénylméthanol.

Dans un bicol de 200 ml, sous atmosphère d'azote, on introduit 2,96 g (78,1 mmoles) d'hydrure double d'aluminium et de lithium dans 50 ml de tétrahydrofurane anhydre, on chauffe le mélange au reflux, on ajoute 4,49 g (15,4 mmoles) d'une solution de (1*S*)-2-[(2*S*)-hydroxy(phényl)méthyl]pipéridine-1-carboxylate de 1,1-diméthyléthyle dans 35 ml de tétrahydrofurane et on maintient le mélange au reflux pendant 3,5 h.
On le refroidit, on l'hydrolyse lentement avec une solution 0,1M de tartrate double de potassium et de sodium et on laisse le mélange sous agitation pendant une nuit.
On le filtre et on rinçe le précipité avec du tétrahydrofurane, puis on concentre le filtrat sous pression réduite.
On obtient 2,95 g d'un produit huileux incolore.

2.4. (1*S*)-[(2*S*)-(1-méthylpipéridin-2-yl)]phénylméthanamine.

Dans un ballon de 250 ml, sous atmosphère d'azote, on introduit 2,95 g (14,4 mmoles) de (1*S*)-[(2*S*)-(1-méthylpipéridin-2-yl)]phénylméthanol et 2 ml (14,4 mmoles) de triéthylamine dans 70 ml de dichlorométhane anhydre, on refroidit le milieu à 0°C, on ajoute 1,1 ml (14,4 mmoles) de chlorure de méthane sulfonyle, on laisse le mélange revenir lentement à température ambiante pendant 2 h et on le concentre sous pression réduite.
Dans un autoclave muni d'une agitation magnétique et refroidi à -50°C on introduit de l'ammoniac liquéfié, on ajoute une solution du méthanesulfonate brut précédemment préparé en solution dans 30 ml d'éthanol absolu, on ferme l'autoclave et on maintient l'agitation pendant 48 h.
On transvase le mélange dans un ballon et on isolé l'amine sous forme de produit huileux qu'on utilise tel quel dans l'étape suivante.

2.5. Chlorhydrate de 2-chloro-*N*-[(1*S*)-[(2*S*)-1-méthylpipéridin-2-yl]phénylméthyl]-3-trifluorométhylbenzamide 1:1.

En utilisant le mode opératoire décrit au point 1.6, à partir de 1 g (4,9 mmoles) d'acide 2-chloro-3-trifluorométhylbenzoïque, 0,9 g (4,9 mmoles) de chlorhydrate de 1-[3-(diméthylamino)propyl]-3-éthylcarbodiimide, 0,66 g (4,6 mmoles) de 1-hydroxybenzotriazole et 1 g (4,9 mmoles) de (1*S*)-[(2*S*)-(1-méthylpipéridin-2-yl)]phénylméthanamine, on obtient, après purification par chromatographie sur colonne de gel de silice en éluant avec un mélange de dichlorométhane et de méthanol, 0,45 g de produit sous forme de base.
On dissout ce dernier dans quelques ml de propan-2-ol, on ajoute 10,9 ml d'une solution 1N d'acide chlorhydrique dans le propan-2-ol et on concentre le mélange sous pression réduite pour diminuer le volume de solvant.
Après trituration on isole finalement 0,37 g de chlorhydrate sous forme d'un solide blanc.
Point de fusion : 230-232°C.

$[\alpha]_D^{25}=+70,3°$ (c=0,825 ; $CH_3OH$) ee>99%.

<u>Exemple 3</u> (Composé N°24).

Chlorhydrate de thréo-4-amino-3-chloro-*N*-[(1-méthylpipéridin-2-yl)phénylméthyl]-5-trifluorométhylbenzamide 1:1.

3.1. Trifluorométhanesulfonate de 2-(benzyloxyiminophénylméthyl)-1-méthylpyridinium.

A une suspension de 35 g (120 mmoles) de phényl(pyridin-2-yl)méthanone O-benzyloxime dans 200 ml d'éther diéthylique on ajoute, goutte à goutte et à 0°C, 17,4 ml (120 mmoles) de trifluorométhane sulfonate de méthyle et on agite le mélange à température ambiante pendant 3 h. On recueille le précipité formé par filtration et on le sèche sous pression réduite.
On obtient 49 g de produit qu'on utilise tel quel dans l'étape suivante.

3.2. Ethanedioate de thréo-(1-méthylpipéridin-2-yl)phénylméthanamine 2:1.

Dans une fiole de Parr on place 14,8 g (31,89 mmoles) de trifluorométhane sulfonate de 2-(benzyloxyiminophénylméthyl)-1-méthylpyridinium et 0,74 g d'oxyde de platine dans 50 ml d'éthanol et 50 ml d'acide chlorhydrique 1N et on effectue une hydrogénation pendant 5 h.
On évapore l'éthanol sous pression réduite, on extrait le résidu avec du dichlorométhane, on sépare la phase aqueuse, on y ajoute une solution d'ammoniaque et on l'extrait avec du dichlorométhane. Après lavage des phases organiques réunies, séchage sur sulfate de sodium, filtration et évaporation du solvant sous pression réduite, on obtient 6,7 g de produit huileux comprenant 10% de diastéréoisomère érythro.
On prépare l'éthanedioate en dissolvant ces 6,7 g de base dans le méthanol, par action de deux équivalents d'acide éthanedioïque dissous dans le minimum de méthanol.
On purifie le sel obtenu par recristallisation dans un mélange de méthanol et d'éther diéthylique.
On isole finalement 4,7 g d'éthanedioate du diastéréoisomère thréo pur.
Point de fusion : 156-159°C.

3.3. Acide 4-amino-3-chloro-5-trifluorométhylbenzoïque.

Dans un ballon de 500 ml on place 7,8 g (40 mmoles) d'acide 4-amino-5-trifluorométhylbenzoïque dans 80 ml de chloroforme en présence de 9,97 ml (50 mmoles) de chlorure de sulfuryle et on agite le mélange au reflux pendant une nuit.
On évapore le solvant sous pression réduite, on reprend le résidu avec de l'eau et de l'ammoniaque et on extrait le mélange avec du dichlorométhane. On acidifie la phase aqueuse, on recueille le précipité formé par filtration et on le sèche sous pression réduite.
On obtient 9 g de produit.
Point de fusion : 229-235°C.

3.4. Chlorhydrate de thréo-4-amino-3-chloro-*N*-[(1-méthylpipéridin-2-yl)phénylméthyl]-5-trifluorométhylbenzamide 1:1.

Dans un ballon de 100 ml on place 0,52 g (2,15 mmoles) d'acide 4-amino-3-chloro-5-trifluorométhylbenzoïque, 0,37 g (1,96 mmole) de chlorhydrate de 1-[3-(diméthylamino)propyl]-3-éthylcarbodiimide, 0,26 g (1,96 mmole) de 1-hydroxybenzotriazole dans 5 ml de 1,2-dichloroéthane et on agite le mélange à température ambiante pendant 10 min.
On ajoute 0,4 g (1,96 mmole) de thréo-(1-méthylpipéridin-2-yl)phénylméthanamine en solution dans 5 ml de 1,2-dichloroéthane et on laisse le mélange sous agitation pendant 12 h.
On l'hydrolyse avec de l'eau, on ajoute de la potasse en pastilles jusqu'à obtenir un pH basique, et on extrait le mélange avec du dichlorométhane. On lave la phase organique à l'eau, on la sèche sur sulfate de sodium, on la filtre, on évapore le solvant sous pression réduite et on purifie le résidu par chromatographie sur colonne de gel de silice en éluant avec un mélange de dichlorométhane et de méthanol.
On isole 0,4 g de composé sous forme de base.
On le dissout dans quelques ml de propan-2-ol, on ajoute 9,4 ml d'une solution 0,1N d'acide chlorhydrique dans le propan-2-ol et on évapore le solvant sous pression réduite. On collecte le résidu et on le sèche sous vide.
On obtient 0,285 g de produit solide.
Point de fusion : 270-272°C.

<u>Exemple 4</u> (Composé N°25).

Chlorhydrate de 4-amino-3-chloro-*N*-[(1*R*)-[(2*R*)-1-méthyl-pipéridin-2-yl]phénylméthyl]-5-trifluorométhylbenzamide 1:1.

4.1. (1*R*)-[(2*R*)-(1-méthylpipéridin-2-yl)]phénylméthanamine.

Dans un ballon de 4 l on introduit 80 g (390 mmoles) de thréo-(1-méthylpipéridin-2-yl)phénylméthanamine en solution dans 300 ml de méthanol et 68 g (390 mmoles) de
*N*-acétyl-D-leucine en solution dans 450 ml de méthanol. On concentre la solution sous pression réduite et on recristallise le résidu dans 1100 ml de propan-2-ol. On obtient 72 g de sels de (1*R*)-[(2*R*)-(1-méthylpipéridin-2-yl)phénylméthanamine.
On réitère trois fois la recristallisation et on obtient finalement 15 g de sel de (1*R*)-[(2*R*)-(1-méthylpipéridin-2-yl]phénylméthanamine.
Point de fusion : 171,5°C.

$[\alpha]_D^{25} = -11°$ (c=1 ; $CH_3OH$) ee>99%.

4.2. Chlorhydrate de 4-amino-3-chloro-*N*-[(1*R*)-[(2*R*)-1-méthylpipéridin-2-yl]phénylméthyl]-5-trifluorométhylbenzamide 1:1.

En utilisant le mode opératoire décrit au point 3.4 ci-dessus, à partir de 1,04 g (4,37 mmoles) d'acide 4-amino-3-chloro-5-trifluorométhylbenzoïque, de 0,46 g (3,97 mmoles) de chlorhydrate de 1-[3-(diméthylamino)propyl]-3-éthylcarbodiimide, de 0,53 g (3,97 mmoles) de 1-hydroxybenzotriazole et de 1,5 g (3,97 mmoles) de (1*R*)-[(2*R*)-méthylpipéridin-2-yl]phénylméthanamine, on obtient 1,12 g de produit sous forme de base.
On en prépare le chlorhydrate en ajoutant 28,2 ml d'une solution 0,1N d'acide chlorhydrique dans le propan-2-ol à une solution de 1,12 g de base en solution dans quelques ml de propan-2-ol. On évapore le solvant sous pression réduite, on collecte le solide obtenu et on le sèche sous pression réduite.
On isole finalement 0,9 g de chlorhydrate sous forme d'un solide blanc.Point de fusion : 175-185°C.

$[\alpha]_D^{25}=+18,4°$ (c=0,091 ; $CH_3OH$) ee=97,8%.

Exemple 5 (Composé N°36).

Chlorhydrate de thréo-2-chloro-*N*-[phényl(pipéridin-2-yl)méthyl]-3-trifluorométhylbenzamide 1:1.

5.1. 2-chloro-*N*-[phényl(pyridin-2-yl)méthyl]-3-trifluorométhylbenzamide.

Dans un ballon de 250 ml on place 1,61 g (7,16 mmoles) d'acide 2-chloro-3-trifluorométhylbenzoïque, 1,4 g (7,28 mmoles) de chlorhydrate de 1-[3-(diméthylamino)propyl]-3-éthylcarbodiimide, 0,218 g (1,79 mmole) de 4-diméthylaminopyridine en solution dans 60 ml de dichlorométhane, on agite le mélange pendant 15 min, on ajoute 1,1 g (5,97 mmoles) de phényl(pyridin-2-yl)méthanamine en solution dans 60 ml de dichlorométhane et on agite le mélange à température ambiante pendant 24 h.
On l'hydrolyse en ajoutant de l'eau, on ajoute une solution de soude aqueuse à 35%, on sépare la phase organique, on la lave à l'eau puis avec une solution aqueuse saturée de chlorure de sodium, on la sèche sur sulfate de magnésium, on la filtre et on évapore le solvant sous pression réduite. On purifie le résidu par chromatographie sur colonne de gel de silice en éluant avec un mélange de dichlorométhane et de méthanol et on isole finalement 1,34 g de produit sous forme d'huile jaune qui cristallise, et qu'on utilise telle quelle dans l'étape suivante.

5.2. Chlorhydrate de thréo-2-chloro-*N*-[phényl(pipéridin-2-yl)méthyl]-3-trifluorométhylbenzamide 1:1.

Dans une fiole de Parr on place une solution de 4,17 g (10 mmoles) de 2-chloro-*N*-[phényl(pyridin-2-yl)méthyl]-3-trifluorométhylbenzamide dans 43 ml d'acide acétique glacial, on ajoute 0,1 g de charbon palladié à 5% et on effectue une hydrogénation sous 0,35 Mpa à 50°C pendant 3 h.
Après retour à la température ambiante on élimine le catalyseur par filtration, on concentre le filtrat sous pression réduite, on reprend le résidu avec de l'eau et de l'acétate d'éthyle, on ajoute de la soude concentrée et on extrait le mélange plusieurs fois avec de l'acétate d'éthyle. On lave la phase organique à l'eau puis avec une solution aqueuse saturée de chlorure de sodium, on la sèche sur sulfate de sodium, on la filtre et on évapore le solvant sous pression réduite. On purifie le résidu par deux chromatographies successives sur colonne de gel de silice en éluant avec un mélange 100/0 à 95/5 de dichlorométhane et de méthanol, pour séparer le produit de départ qui n'a pas réagi.
On isole 0,8 g du diastéréoisomère thréo (moins polaire).
On en prépare le chlorhydrate en le dissolvant dans quelques ml de propan-2-ol et en y ajoutant 20 ml d'une solution 0,1N d'acide chlorhydrique dans le propan-2-ol. On évapore partiellement le solvant sous pression réduite, on obtient un solide blanc par trituration, on le collecte par filtration et on le sèche sous pression réduite.
On obtient finalement 0,6 g de chlorhydrate.
Point de fusion : 234-235°C.

Exemple 6 (Composé N°37).

Chlorhydrate de 2-chloro-*N*-[(*S*)-phényl-[(2*S*)-pipéridin-2-yl]méthyl]-3-(trifluorométhyl)benzamide 1:1.

Dans un bicol de 500 ml muni d'une agitation magnétique, d'une circulation d'argon et d'un réfrigérant, on introduit 8,36 g (3 eq.) d'acide 1,3-diméthylbarbiturique en solution dans 100 ml de dichlorométhane anhydre. On ajoute 0,2 g (0,01 eq.) de palladium tétrakistriphénylphosphine et on chauffe le milieu-réactionnel à 35°C. On ajoute une solution de 7,8 g (19,18 mmoles) de *N*-[(*S*)-[(2-*S*)-1-allylpipéridin-2-yl](phényl)méthyl]-2-chloro-3-((trifluorométhyl)benzamide (obtenu selon un mode opératoire analogue à celui de l'exemple 1), et on suit l'évolution de la réaction par chromatographie sur couche mince. On ajoute 100 ml d'une solution saturée d'hydrogénocarbonate de sodium, on décante et on extrait la phase aqueuse deux fois avec 100 ml de dichlorométhane, on lave les phases organiques réunies avec 100 ml d'eau puis 100 ml d'une solution saturée de chlorure de sodium. On sèche sur sulfate de sodium, on filtre et on évapore le solvant sous pression réduite.
On obtient 10,15 g de solide beige que l'on purifie par chromatographie sur colonne de gel de silice en éluant avec un mélange de dichlorométhane contenant 0,4% d'une solution d'ammoniac à 33%.
On isole 4,8 g de solide blanchâtre. On dissout ce dernier dans 50 ml de propan-2-ol et on ajoute 125 ml d'une solution 0,1N d'acide chlorhydrique dans le propan-2-ol et on concentre le mélange sous pression réduite pour diminuer le volume de solvant.
Après trituration on isole 4,33 g de chlorhydrate sous forme de cristaux blancs.
Point de fusion : 223-225°C.

$[\alpha]_D^{25} = +80,7°$ (c=0,5 ; $CH_3OH$) ee>98%.

Exemple 7 (Composés N°69 et 70).

2-Chloro-*N*-[[1-méthyl-1-oxido-pipéridin-2-yl](phényl)méthyl]-3-trifluorométhylbenzamide.

Dans un ballon de 50 ml muni d'une agitation magnétique, on introduit 0,54 g (1,3 mmole) de *thréo*-2-chloro-*N*-[(1-méthylpipéridin-2-yl)phénylméthyl]-3-trifluorométhylbenzamide dans 20 ml de dichlorométhane anhydre à 0°C, on ajoute une solution de 0,28 g (1,2 eq.) d'acide 3-chloroperbenzoïque dans 5 ml de dichlorométhane et on laisse revenir à température ambiante sous agitation pendant 12 h.
On ajoute 30 ml d'eau, on décante et on extrait la phase aqueuse deux fois avec 30 ml de dichlorométhane, on lave les phases réunies avec 100 ml d'eau puis 100 ml d'une solution saturée de chlorure de sodium. On sèche la pfase organique sur sulfate de sodium, et on élimine les solvants sous pression réduite et on purifie le résidu par chromatographie sur colonne de gel de silice en éluant avec un mélange de 90/10 de dichlorométhane et de méthanol en 40 min. On isole 0,15 g du premier isomère *N*-oxyde (Point de fusion : 100-102°C) et 0,03 g du second isomère *N*-oxyde (Point de fusion : 126-128°C).

Exemple 8 (Composé N°71).

Iodure de (2*S*)-2[(1*S*)-[[2-chloro-3-(trifluorométhyl)benzoyl]amino](phényl)méthyl]-1,1-diméthylpipéridinium.

Dans un bicol de 50 ml muni d'une agitation magnétique, d'une circulation d'argon et d'un réfrigérant, on introduit 0,15 g (0,36 mmole) de 2-chloro-*N*-[(1*S*)-[(2*S*)-1-méthylpipé_ ridin-2-yl]phénylméthyl]-3-trifluorométhylbenzamide en solution dans 20 ml d'acétonitrile, on ajoute 0,5 ml d'iodométhane et on chauffe à 80°C pendant 2 h.
On concentre de moitié le milieu réactionnel, le sel d'ammonium précipite, on le filtre et on le sèche sous pression réduite.
On isole 0,17 g de solide jaune.
Point de fusion : 121-123°C.

Le tableau 1 qui suit illustre les structures chimiques de quelques composés de l'invention.
Dans la colonne "A", $cC_3H_5$ désigne un groupe cyclopropyle. Dans la colonne "$CF_3$" est indiquée la position du groupe $CF_3$ dans la formule générale (I). Dans la colonne "$R_2$", $C_6H_5$ désigne un groupe phényle. Dans la colonne "Sel", "-" désigne un composé à l'état de base, "HCl" désigne un chlorhydrate et "tfa" désigne un trifluoroacétate.
Le tableau 2 illustre les propriétés physiques, points de fusion et pouvoirs rotatoires, de quelques composés.

Tableau 1

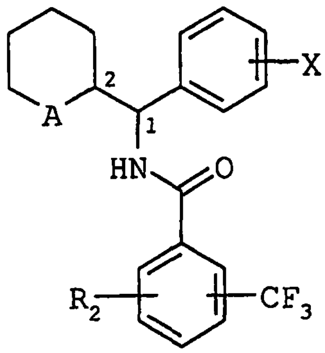

| N° | stéréochimie | A | X | $CF_3$ | $R_2$ | Sel |
|---|---|---|---|---|---|---|
| 1 | thréo (1*R*,2*R*;1*S*,2*S*) | $N\text{-}CH_3$ | H | 6 | 2-F, 3-Cl | HCl |
| 2 | thréo (1*R*,2*R*;1*S*,2*S*) | $N\text{-}CH_3$ | H | 2 | $4\text{-}CF_3$ | HCl |
| 3 | thréo (1*R*,2*R*;1*S*,2*S*) | $N\text{-}CH_3$ | H | 2 | $6\text{-}CF_3$ | HCl |
| 4 | thréo (1*R*,2*R*;1*S*,2*S*) | $N\text{-}CH_3$ | H | 2 | 5-Cl | HCl |
| 5 | thréo (1*R*,2*R*;1*S*,2*S*) | $N\text{-}CH_3$ | H | 2 | 4-F | - |
| 6 | thréo (1*R*,2*R*;1*S*,2*S*) | $N\text{-}CH_3$ | H | 2 | $5\text{-}CF_3$ | - |
| 7 | thréo (1*R*,2*R*;1*S*,2*S*) | $N\text{-}CH_3$ | H | 2 | 3-Cl | HCl |
| 8 | thréo (1*R*,2*R*;1*S*,2*S*) | $N\text{-}CH_3$ | H | 4 | $2,6\text{-}Cl_2$ | HCl |
| 9 | thréo (1*R*,2*R*;1*S*,2*S*) | $N\text{-}CH_3$ | H | 4 | 2-Cl | HCl |
| 10 | thréo (1*R*,2*R*;1*S*,2*S*) | $N\text{-}CH_3$ | H | 4 | 3-Cl | HCl |
| 11 | thréo (1*R*,2*R*;1*S*,2*S*) | $N\text{-}CH_3$ | H | 3 | 4-F | HCl |
| 12 | thréo (1*R*,2*R*;1*S*,2*S*) | $N\text{-}CH_3$ | H | 3 | H | HCl |
| 13 | thréo (1*R*,2*R*;1*S*,2*S*) | $N\text{-}CH_3$ | H | 5 | 2-Cl | HCl |
| 14 | (1*S*,2*S*) | $N\text{-}CH_3$ | H | 5 | 2-Cl | HCl |
| 15 | (1*R*,2*R*) | $N\text{-}CH_3$ | H | 5 | 2-Cl | HCl |
| 16 | thréo (1*R*, 2*R*; 1*S*, 2*S*) | $N\text{-}CH_3$ | H | 3 | $5\text{-}CF_3$ | HCl |
| 17 | thréo (1*R*, 2*R*; 1*S*, 2*S*) | $N\text{-}CH_3$ | H | 3 | 2-Cl | HCl |
| 18 | (1*S*,2*S*) | $N\text{-}CH_3$ | H | 3 | 2-Cl | HCl |
| 19 | (1*R*,2*R*) | $N\text{-}CH_3$ | H | 3 | 2-Cl | HCl |
| 20 | thréo (1*R*,2*R*;1*S*,2*S*) | $N\text{-}CH_3$ | H | 3 | 4-Cl | HCl |
| 21 | thréo (1*R*,2*R*;1*S*,2*S*) | $N\text{-}CH_3$ | H | 5 | 2-F, 3-Cl | - |
| 22 | thréo (1*R*,2*R*;1*S*,2*S*) | $N\text{-}CH_3$ | H | 5 | 2-F | - |
| 23 | thréo (1*R*,2*R*;1*S*,2*S*) | $N\text{-}CH_3$ | H | 5 | $2\text{-}OCH_3, 4\text{-}C_6H_5$ | HCl |
| 24 | thréo (1*R*,2*R*;1*S*,2*S*) | $N\text{-}CH_3$ | H | 5 | $3\text{-}Cl, 4\text{-}NH_2$ | HCl |
| 25 | (1*R*,2*R*) | $N\text{-}CH_3$ | H | 5 | $3\text{-}Cl, 4\text{-}NH_2$ | HCl |
| 26 | thréo (1*R*,2*R*;1*S*,2*S*) | $N\text{-}CH_3$ | $2\text{-}CH_3$ | 3 | 2-Cl | HCl |
| 27 | thréo (1*R*,2*R*;1*S*,2*S*) | $N\text{-}CH_3$ | H | 3 | $2,6\text{-}Cl_2$ | HCl |
| 28 | (1*S*,2*S*) | $N\text{-}CH_3$ | H | 3 | $2,6\text{-}Cl_2$ | HCl |
| 29 | thréo (1*R*,2*R*;1*S*,2*S*) | $N\text{-}CH_3$ | 4-F | 3 | 2-Cl | HCl |
| 30 | (1*S*,2*S*) | $N\text{-}CH_3$ | 4-F | 3 | 2-Cl | HCl |
| 31 | (1*S*,2*S*) | $N\text{-}CH_3$ | 4-Cl | 3 | 2-Cl | HCl |
| 32 | (1*S*,2*S*) | $N\text{-}CH_3$ | $4\text{-}C(CH_3)$ | 3 | 2-Cl | tfa |
| 33 | thréo (1*R*,2*R*;1*S*,2*S*) | $N\text{-}CH_2CH_3$ | H | 3 | 2-Cl | HCl |
| 34 | (1*S*,2*S*) | $N\text{-}CH_3$ | $4\text{-}CH_3$ | 3 | 2-Cl | HCl |
| 35 | thréo (1*R*,2*R*;1*S*,2*S*) | $N\text{-}CH_3$ | H | 2 | 4-Cl | HCl |
| 36 | thréo (1*R*,2*R*;1*S*,2*S*) | NH | H | 3 | 2-Cl | HCl |

(suite)

| N° | stéréochimie | A | X | $CF_3$ | $R_2$ | Sel |
|---|---|---|---|---|---|---|
| 37 | (1*S*,2*S*) | NH | H | 3 | 2-Cl | HCl |
| 38 | (1*R*,2*R*) | NH | H | 3 | 2-Cl | HCl |
| 39 | thréo (1*R*,2*R*;1*S*,2*S*) | $N-CH_2CH(CH_3)_2$ | H | 3 | 2-Cl | HCl |
| 40 | (1*S*,2*S*) | $N-CH_2CH(CH_3)_2$ | H | 3 | 2-Cl | HCl |
| 41 | thréo (1*R*,2*R*;1*S*,2*S*) | $N-(CH_2)_2CH_3$ | H | 3 | 2-Cl | HCl |
| 42 | (1*S*,2*S*) | $N-(CH_2)_2CH_3$ | H | 3 | 2-Cl | HCl |
| 43 | (1*S*,2*S*) | $N-CH_2cC_3H_5$ | H | 3 | 2-Cl | HCl |
| 44 | thréo (1*R*,2*R*;1*S*,2*S*) | $N-CH_3$ | H | 3 | $2-CH_3$ | HCl |
| 45 | (1*S*,2*S*) | $N-CH(CH_3)_2$ | H | 3 | 2-Cl | HCl |
| 46 | (1*S*,2*S*) | $N-(CH_2)_3CH_3$ | H | 3 | 2-Cl | HCl |
| 47 | (1*S*,2*S*) | $N-CH_2C{=}CH$ | H | 3 | 2-Cl | HCl |
| 48 | (1*S*,2*S*) | $N-CH_2C_6H_5$ | H | 3 | 2-Cl | HCl |
| 49 | (1*S*,2*S*) | $N-CH_2[3,4-(OCH_3)_2C_6H_3]$ | H | 3 | 2-Cl | - |
| 50 | thréo (1*R*,2*R*;1*S*,2*S*) | $N-CH_3$ | H | 5 | $2-CH_3$ | HCl |
| 51 | thréo (1*R*,2*R*;1*S*,2*S*) | $N-(CH_2)_3CF_3$ | H | 3 | 2-Cl | HCl |
| 52 | (1*S*,2*S*) | $N-(CH_2)_2CH_3$ | H | 3 | $2-CH_3$ | HCl |
| 53 | thréo (1*R*,2*R*;1*S*,2*S*) | $N-(CH_2)_2CH_3$ | 4-F | 3 | $2-CH_3$ | HCl |
| 54 | thréo (1*R*,2*R*;1*S*,2*S*) | $N-(CH_2)_2CH_3$ | 4-F | 3 | 2-Cl | HCl |
| 55 | thréo (1*R*,2*R*;1*S*,2*S*) | $N-(CH_2)_2CH_3$ | 4-Cl | 3 | 2-Cl | HCl |
| 56 | thréo (1*R*,2*R*;1*S*,2*S*) | $N-(CH_2)_2CH_3$ | 4-Cl | 3 | $2-CH_3$ | HCl |
| 57 | (1*S*,2*S*) | $N-CH_3$ | H | 3 | $2-CH_3$ | HCl |
| 58 | (1*S*,2*S*) | $N-(CH_2)_2CH_3$ | 4-F | 3 | 2-Cl | HCl |
| 59 | thréo (1*R*,2*R*;1*S*,2*S*) | $N-CH_2CH{=}CH_2$ | H | 3 | 2-Cl | HCl |
| 60 | (1*S*,2*S*) | $N-CH_2CH{=}CH_2$ | H | 3 | 2-Cl | HCl |
| 61 | (1*S*,2*S*) | NH | H | 3 | $2-CH_3$ | HCl |
| 62 | (1*S*,2*S*) | NH | H | 6 | 2-F, 3-Cl | HCl |
| 63 | (1*S*,2*S*) | NH | H | 5 | 2-Cl | HCl |
| 64 | thréo (1*R*,2*R*;1*S*,2*S*) | NH | H | 2 | $4-CF_3$ | HCl |
| 65 | thréo (1*R*,2*R*;1*S*,2*S*) | NH | H | 3 | H | HCl |
| 66 | thréo (1*R*,2*R*;1*S*,2*S*) | NH | H | 3 | 2-F | HCl |
| 67 | thréo (1*R*,2*R*;1*S*,2*S*) | NH | H | 3 | $5-CF_3$ | HCl |
| 68 | thréo (1*R*,2*R*;1*S*,2*S*) | NH | H | 2 | $5-CF_3$ | HCl |
| 69 | thréo (1*R*,2*R*;1*S*,2*S*) | $N^+(O^-)CH_3$ | H | 3 | 2-Cl | HCl |
| 70 | thréo (1*R*,2*R*;1*S*,2*S*) | $N^+(O^-)CH_3$ | H | 3 | 2-Cl | HCl |
| 71 | (1*S*,2*S*) | $N^+(CH_3)_2$ | H | 3 | 2-Cl | HCl |
| Composé N°69 : diastéréoisomère le plus polaire<br>Composé N°70 : diastéréoisomère le moins polaire | | | | | | |

Tableau 2

| N° | F (°C) | $[\alpha]_D^{25}$ |
|---|---|---|
| 1 | >270 | - |
| 2 | 152-154 | - |
| 3 | >285 | - |
| 4 | 275-276 | - |
| 5 | 51-52 | - |
| 6 | 169 | - |
| 7 | 228-229 | - |

(suite)

| N° | F (°C) | $[\alpha]_D^{25}$ |
|---|---|---|
| 8 | 287-288 | - |
| 9 | 84-86 | - |
| 10 | 187-191 | - |
| 11 | 237,5-238,5 | - |
| 12 | 174-176 | - |
| 13 | 229-231 | - |
| 14 | 95-100 | +67,7 (c=0,26 ; $CH_3OH$) |
| 15 | 95-100 | -66,5 (c=0,275 ; $CH_3OH$) |
| 16 | 200-201,5 | - |
| 17 | 215-216 | - |
| 18 | 230-232 | +70, 7 (c=0,825 ; $CH_3OH$) |
| 19 | 243-248 | -74,26 (c=0,715 ; $CH_3OH$) |
| 20 | 225-227 | - |
| 21 | 150-151 | - |
| 22 | 196-197 | - |
| 23 | 153-154 | - |
| 24 | 270-272 | - |
| 25 | 175-185 | +18,4 (c=0,091 ; $CH_3OH$) |
| 26 | 277-279 | - |
| 27 | 297-300 | - |
| 28 | 260-262 | +50, 53 (c=0, 56 ; $CH_3OH$) |
| 29 | 109-111 | - |
| 30 | 236-238 | +50,23 (c=0,325 ; $CH_3OH$) |
| 31 | 238-240 | |
| 32 | 95-97 | |
| 33 | 230-232 | - |
| 34 | 222-224 | +70,9 (c=0,573 ; $CH_3OH$) |
| 35 | 258-259 | - |
| 36 | 234-235 | - |
| 37 | 223-225 | +80, 7 (c=0,5 ; $CH_3OH$) |
| 38 | 217-219 | -74,2 (c=0,51 ; $CH_3OH$) |
| 39 | 158-160 | - |
| 40 | 80-82 | +67,3 (c=0,854 ; $CH_3OH$) |
| 41 | 124-126 | - |
| 42 | 210-212 | +80, 7 (c=0,896 ; $CH_3OH$) |
| 43 | 200-202 | +71, 7 (c=0, 882 ; $CH_3OH$) |
| 44 | 259-260 | - |
| 45 | 256-258 | +18, 1 (c=1 ; $CH_3OH$) |
| 46 | 200-202 | +79,7 (c=0,798 ; $CH_3OH$) |
| 47 | 79-81 | - |
| 48 | 216-218 | +66,4 (c=1 ; $CH_3OH$) |
| 49 | 132 | |
| 50 | 256-257 | |
| 51 | 162-164 | |
| 52 | 101-103 | +57,9 (c=0,87 ; $CH_3OH$) |
| 53 | 234-236 | |
| 54 | 110-112 | |
| 55 | 199-201 | |
| 56 | 94-96 | |

(suite)

| N° | F (°C) | $[\alpha]_D^{25}$ |
|---|---|---|
| 57 | 141-143 | +56,3 (c=0,59 ; $CH_3OH$) |
| 58 | 224-226 | +74,90(c=0,66 ; $CH_3OH$) |
| 59 | 138-140 | |
| 60 | 104-106 | +78,5 (c=0,57 ; $CH_3OH$) |
| 61 | 214-216 | +54,8 (c=0,2 ; $CH_3OH$) |
| 62 | 135-137 | +86,3 (c=0,5 ; $CH_3OH$) |
| 63 | 194-196 | +61,5 (c=0,5 ; $CH_3OH$) |
| 64 | 149-151 | |
| 65 | 199-201 | |
| 66 | 221-223 | |
| 67 | 167-169 | |
| 68 | 255-257 | |
| 69 | 126-128 | |
| 70 | 100-102 | |
| 71 | 121-123 | |

Les composés de l'invention ont été soumis à une série d'essais pharmacologiques qui ont mis en évidence leur intérêt comme substances à activités thérapeutiques.

Etude du transport de la glycine dans les cellules SK-N-MC exprimant le transporteur humain glyt1 natif.

La capture de $[^{14}C]$glycine est étudiée dans les cellules SK-N-MC (cellules neuro-épithéliales humaines) exprimant le transporteur humain glyt1 natif par la mesure de la radioactivité incorporée en présence ou en absence du composé à tester. Les cellules sont cultivées en monocouche pendant 48 h dans des plaques prétraitées à la fibronectine à 0,02%. Le jour de l'expérience, le milieu de culture est éliminé et les cellules sont lavées par un tampon Krebs-HEPES (acide [4-(2-hydroxyéthyl)pipérazine-1-éthanesulfonique) à pH 7,4. Après 10 min de préincubation à 37°C en présence soit de tampon (lot témoin), soit de composé à tester a différentes concentrations ou de 10 mM de glycine (détermination de la capture non spécifique), 10 µM de $[^{14}C]$glycine (activité spécifique 112 mCi/mmole) sont ensuite ajoutés. L'incubation se poursuit pendant 10 min à 37°C, et la réaction est arrêtée par 2 lavages avec un tampon Krebs-HEPES à pH 7,4. La radioactivité incorporée par les cellules est alors estimée après ajout de 100 µl de scintillant liquide et agitation pendant 1 h. Le comptage est réalisé sur compteur Microbeta Tri-lux™. L'efficacité du composé est déterminée par la $CI_{50}$, concentration du composé qui diminue de 50% la capture spécifique de glycine, définie par la différence de radioactivité incorporée par le lot témoin et le lot qui a reçu la glycine à 10 mM.
Les composés de l'invention, dans ce test, ont une $CI_{50}$ de l'ordre de 0,0001 à 10 µM.

Etude *ex vivo* de l'activité inhibitrice d'un composé sur la capture de la $[^{14}C]$glycine dans l'homogénat cortical de souris.

Des doses croissantes du composé à étudier sont administrées par voie orale (préparation par trituration de la molécule à tester dans un mortier dans une solution de Tween/Methocel™ à 0,5% dans de l'eau distillée) ou intrapéritonéale (dissolution de la molécule à tester dans du sérum physiologique ou prépation par trituration dans un mortier dans une solution de Tween/methocel à 0,5% dans de l'eau, selon la solubilité de la molécule) sur des souris mâles OF1 Iffa Crédo de 20 à 25 g le jour de l'expérience. Le groupe témoin est traité par le véhicule. Les doses en mg/kg, la voie d'administration et le temps de traitement sont déterminés en fonction de la molécule à étudier.
Après euthanasie par décapitation des animaux à un temps donné après l'administration, le cortex de chaque animal est rapidement prélevé sur glace, pesé.et conservé à 4°C ou congelé à -80°C (dans les deux cas les échantillons sont conservés 1 jour maximum). Chaque échantillon est homogénéisé dans un tampon Krebs-HEPES à pH 7,4 à raison de 10 ml/g de tissu. 20 µl de chaque homogénat sont incubés pendant 10 min à température ambiante en présence de 10 mM de L-alanine et de tampon. La capture non spécifique est déterminée par addition de 10 mM de glycine au groupe témoin. La réaction est arrêtée par filtration sous vide et la radioactivité retenue est estimée par scintillation solide par comptage sur compteur Microbeta Tri-lux™.
Un inhibiteur de la capture de la $[^{14}C]$glycine diminuera la quantité de radioligand incorporée dans chaque homogénat. L'activité du composé est évaluée par sa $DE_{50}$, dose qui inhibe 50% de la capture de la $[^{14}C]$glycine par rapport au

groupe témoin.
Les composés de l'invention les plus puissants, dans ce test, ont une $DE_{50}$ de 0,1 à 5 mg/kg par voie intrapéritonéale ou par voie orale.

Etude du transport de la glycine dans l'homogénat de moelle épinière de souris.

La capture de [$^{14}$C] glycine par le transporteur glyt2 est étudiée dans l'homogénat de moelle épinière de souris par la mesure de radioactivité incorporée en présence ou en absence de composé à étudier.
Après euthanasie des animaux (souris mâles OF1 Iffa Crédo pesant 20 à 25 g le jour de l'expérience), la moelle épinière de chaque animal est rapidement prélevée, pesée et conservée sur glace. Les échantillons sont homogénéisés dans un tampon Krebs-HEPES (acide [4-(2-hydroxyéthyl)pipérazine-1-éthanesulfonique), pH 7,4, à raison de 25 ml/g de tissu. 50 µl d'homogénat sont pré-incubés pendant 10 min à 25°C en présence de tampon Krebs-HEPES , pH 7,4 et de composé à étudier à différentes concentrations, ou de 10 mM de glycine pour déterminer la capture non spécifique. La [$^{14}$C]glycine (activité spécifique = 112mCi/mmole) est ensuite ajoutée pendant 10 min à 25°C à la concentration finale de 10 µM. La réaction est arrêtée par filtration sous vide et la radioactivité est estimée par scintillation solide par comptage sur un compteur Microbeta Tri-lux™. L'efficacité du composé est déterminée par la concentration $CI_{50}$ capable de diminuer de 50% la capture spécifique de glycine, définie par la différence de radioactivité incorporée par le lot témoin et le lot qui a reçu la glycine 10 mM.
Les composés de l'inventiondans ce test, ont une $CI_{50}$ de l'ordre de 0,0001 à 10 µM.

Etude *ex vivo* de l'activité inhibitrice d'un composé sur la capture de la [$^{14}$C]glycine dans l'homogénat spinal de souris.

Des doses croissantes du composé à étudier sont aministrées par voie orale (préparation par trituration du composé à tester dans un mortier, dans une solution de Tween/Methocel™ à 0,5% dans de l'eau distillée) ou intrapéritonéale (composé à tester dissous dans du sérum physiologique, ou trituré dans un mortier, dans une solution de Tween/Methocel™ à 0,5% dans de l'eau distillée) à des souris mâles OF1 Iffa Crédo de 20 à 25 g le jour de l'expérience. Le groupe témoin est traité par le véhicule. Les doses en mg/kg, la voie d'administration, le temps de traitement ainsi que le temps d'euthanasie sont déterminés en fonction du composé à étudier.
Après euthanasie par décapitation des animaux à un temps donné après l'administration, les moelles sont prélevées rapidement, pesées et introduites dans des fioles à scintillation en verre, conservées dans de la glace pilée ou congelées à -80°C (dans les deux cas les échantillons sont conservés 1 jour maximum). Chaque échantillon est homogénéisé dans un tampon Krebs-HEPES à pH 7,4, à raison de 25 ml/g de tissu. 50 µl de chaque homogénat sont incubés pendant 10 min à température ambiante en présence de tampon.
La capture non spécifique est déterminée par addition de 10 mM de glycine au groupe témoin.
La réaction est arrêtée par filtration sous vide et la radioactivité est estimée par scintillation solide par comptage sur un compteur Microbeta Tri-lux™.
Un inhibiteur de la capture de la [$^{14}$C]glycine diminuera la quantité de radioligand incorporée dans chaque homogénat.
L'activité du composé est évaluée par sa $DE_{50}$, dose efficace qui inhibe 50% de la capture de la [$^{14}$C]glycine par rapport au groupe témoin.
Les meilleurs composés de l'invention ont, dans ce test, une $DE_{50}$ de 1 à 20 mg/kg, par voie intrapéritonéale ou par voie orale.

Les résultats des essais effectués sur les composés de l'invention de configuration (1*S*,2*S*) et leurs racémates thréo de configuration (1*R*,2*R* ;1*S*,2*S*) dans la formule générale (I) desquels $R_2$ représente un ou plusieurs atomes d'halogènes ou groupes trifluorométhyle montrent qu'ils sont des inhibiteurs du transporteur de la glycine glyt1 présents dans le cerveau, et cela *in vitro* et *ex vivo*.

Ces résultats suggèrent que les composés de l'invention peuvent être utilisés pour le traitement des troubles comportementaux associés à la démence, des psychoses, en particulier de la schizophrénie (forme déficitaire et forme productive) et des symptômes extrapyramidaux aigus ou chroniques induits par les neuroleptiques, pour le traitement des diverses formes d'anxiété, des attaques de panique, des phobies, des troubles obsessionnels compulsifs, pour le traitement des différentes formes de dépression, y compris la dépression psychotique, pour le traitement des troubles dus à l'abus ou au sevrage d'alcool, des troubles du comportement sexuel, des troubles de la prise de nourriture, et pour le traitement de la migraine.

Les résultats des essais effectués sur les composés de l'invention de configuration (1*R*,2*R*) et leurs racémates de configuration (1*R*,2*R*;1*S*,2*S*) dans la formule générale (I) desquels $R_2$ représente à la fois un atome d'halogène et un groupe amino $NR_3R_4$ montrent qu'ils sont des inhibiteurs du transporteur des la glycine glyt2, présent majoritairement dans la moelle épinière, et cela *in vitro* et *ex vivo.*

Ces résultats suggèrent que les composés de l'invention peuvent être utilisés pour le traitement des contractures musculaires douloureuses en rhumatologie et en pathologie rachidienne aiguë, pour le traitement des contractures

spastiques d'origine médullaire ou cérébrale, pour le traitement symptomatique des douleurs aiguës et subaiguës d'intensité légère à modérée, pour le traitement des douleurs intenses et/ou chroniques, des douleurs neurogènes et algies rebelles, pour le traitement de la maladie de Parkinson et des symptômes parkinsoniens d'origine neurodégénérative ou induits par des neuroleptiques, pour le traitement des épilepsies généralisées primaires et secondaires, partielles à symptomatologie simple ou complexe, des formes mixtes et autres syndromes épileptiques en complément d'un autre traitement antiépileptique, ou en monothérapie, pour le traitement des apnées du sommeil, et pour la neuroprotection.

C'est pourquoi la présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec des excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.

Lesdites formes unitaires sont dosées pour permettre une administration journalière de 0,01 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.

Les techniques de réalisation peuvent être la compression directe, la granulation sèche, la granulation humide ou la fusion à chaud.

Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.

Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de même qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

**1.** Composé, sous forme d'isomère optique pur (1*R*,2*R*) ou (1S,2S) ou sous forme de diastéréoisomère thréo, répondant à la formule générale (I)

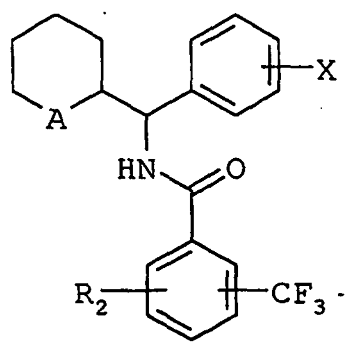

(I)

dans laquelle A représente

soit un groupe de formule générale N-$R_1$ dans laquelle $R_1$ représente soit un atome d'hydrogène, soit un groupe ($C_1$-$C_7$)alkyle linéaire ou ramifié éventuellement substitué par un ou plusieurs atomes de fluor, soit un groupe ($C_4$-$C_7$) cycloalkyle, soit un groupe ($C_3$-$C_7$)cycloalkyl-($C_1$-$C_3$)alkyle, soit un groupe phényl($C_1$-$C_3$)alkyle éventuellement substitué par un ou deux groupes hydroxy ou méthoxy, soit un groupe ($C_2$-$C_4$)alcényle, soit un groupe ($C_2$-$C_4$) alcynyle,

soit un groupe de formule générale $N^+(O^-)R_1$ dans laquelle $R_1$ est tel que défini ci-dessus,

soit encore un groupe de formule générale $N^+(R')R_1$ dans laquelle R' représente un groupe ($C_1$-$C_7$)alkyle linéaire ou ramifié et $R_1$ est tel que défini ci-dessus,

X représente un atome d'hydrogène ou un ou plusieurs substituants choisis parmi les atomes d'halogènes et les groupes trifluorométhyle, ($C_1$-$C_4$)alkyle linéaire ou ramifié et ($C_1$-$C_4$)alcoxy,

$R_2$ représente soit un atome d'hydrogène, soit un ou plusieurs substituants choisis parmi les atomes d'halogènes et les groupes trifluorométhyle, ($C_1$-$C_4$)alkyles, ($C_1$-$C_4$)alcoxy, amino de formule générale $NR_3R_4$ dans laquelle $R_3$ et $R_4$ représentent chacun, indépendamment l'un de l'autre, un atome d'hydrogène ou un groupe ($C_1$-$C_4$)alkyle, ou forment avec l'atome d'azote qui les porte un cycle pyrrolidine, pipéridine ou morpholine, soit un groupe phényle éventuellement substitué par un atome ou un groupe tels que définis pour le symbole X ci-dessus,

à l'état de base libre ou de sel d'addition à un acide.

**2.** Composé selon la revendication 1, **caractérisé en ce que** A représente un groupe de formule générale N-$R_1$ dans laquelle $R_1$ représente soit un atome d'hydrogène, soit un groupe ($C_1$-$C_7$)alkyle linéaire ou ramifié éventuellement substitué par un ou plusieurs atomes de fluor, soit un groupe ($C_4$-$C_7$)cycloalkyle, soit un groupe ($C_3$-$C_7$)cycloalkyl-($C_1$-$C_3$)alkyle, soit un groupe phényl($C_1$-$C_3$)alkyle éventuellement substitué par un ou deux groupes hydroxy ou méthoxy, soit un groupe ($C_2$-$C_4$)alcényle, soit un groupe ($C_2$-$C_4$)alcynyle.

**3.** Composé selon la revendication 1, **caractérisé en ce qu'**il est de configuration (1S,2S) et **en ce que** $R_2$ représente un ou plusieurs atomes d'halogènes ou groupes trifluorométhyle.

**4.** Composé selon la revendication 1, **caractérisé en ce qu'**il est de configuration (1*R*,2*R*) et **en ce que** $R_2$ représente un atome d'halogène et un groupe amino de formule générale $NR_3R_4$ tel que défini dans la revendication 1.

**5.** Composé selon la revendication 1, **caractérisé en ce qu'**il est choisi parmi les composés suivants :

- Chlorhydrate de thréo-2-Chloro-*N*-[(1-éthylpiperidin-2-yl)phénylméthyl]-3-trifluorométhylbenzamide ;
- Chlorhydrate de 2-Chloro-*N*-[(1*S*)-[(2*S*)-1-méthylpipéridin-2-yl]phénylméthyl]-3-trifluorométhylbenzamide ;
- 2-Chloro-*N*-[(1*S*)-[(2*S*)-1-méthylpipéridin-2-yl]phénylméthyl]-3-trifluorométhylbenzamide ;
- Chlorhydrate de thréo-4-Amino-3-chloro-*N*-[(1-méthylpipéridin-2-yl)phénylméthyl]-5-trifluorométhylbenzamide ;
- Thréo-4-Amino-3-chloro-*N*-[(1-méthylpipéridin-2-yl)phénylméthyl]-5-trifluorométhylbenzamide ;
- Chlorhydrate de 4-Amino-3-chloro-*N*-[(1*R*)-[(2*R*)-1-méthylpipéridin-2-yl]phénylméthyl]-5-trifluorométhylbenzamide ;
- 4-Amino-3-chloro-*N*-[(1*R*)-[(2*R*)-1-méthylpiperidin-2-yl]phénylméthyl]-5-trifluorométhylbenzamide ;
- Chlorhydrate de thréo-2-Chloro-*N*-[phényl(pipéridin-2-yl)méthyl]-3-trifluorométhylbenzamide ;
- Chlorhydrate de 2-Chloro-*N*-((*S*)-phényl-[(2*S*)-pipéridin-2-yl]méthyl]-3-(trifluorométhyl)benzamide ;
- 2-Chloro-*N*-[(*S*)-phényl-[(2*S*)-pipéridin-2-yl]méthyl]-3-(trifluorométhyl)benzamide
- 2-Chloro-*N*-[[1-méthyl-1-oxido-pipéridin-2-yl](phényl)méthyl]-3-trifluorométhylbenzamide ;
- Iodure de (2*S*)-2[(1*S*)-[(2-Chloro-3-(trifluorométhyl)benzoyl]amino](phényl)méthyl]-1,1-diméthylpipéridinium.

**6.** Composé selon la revendication 1, **caractérisé en ce qu'** il est choisi parmi le 2-Chloro-*N*-[(S)-phényl-[(2*S*)-pipéridin-2-yl]méthyl]-3-(trifluorométhyl)benzamide ou son sel de chlorhydrate.

**7.** Médicament **caractérisé en ce qu'**il consiste en un composé selon l'une des revendications 1 à 6.

**8.** Composition pharmaceutique **caractérisée en ce qu'**elle contient un composé selon l'une des revendications 1 à 6, associé à un excipient.

**9.** Utilisation d'un composé selon la revendication 1 pour la préparation d'un médicament destiné au traitement des troubles comportementaux liés à la démence, des psychoses, de la schizophrénie (forme déficitaire et forme productive), pour le traitement des diverses formes d'anxiété, pour le traitement des différentes formes de dépression y compris la dépression psychotique, pour le traitement des troubles dus à l'abus d'alcool ou au sevrage d'alcool, des troubles du comportement sexuel, des troubles de la prise de nourriture, pour le traitement de la migraine, pour le traitement des douleurs intenses et/ou chroniques, pour le traitement de la maladie de Parkinson, pour le traitement des épilepsies et autres syndromes épileptiques en complément d'un autre traitement antiépileptique.

## Claims

**1.** Compound, in the form of a pure optical isomer (1*R*,2*R*) or (1*S*,2*S*) or in the form of a threo diastereoisomer, corresponding to general formula (I)

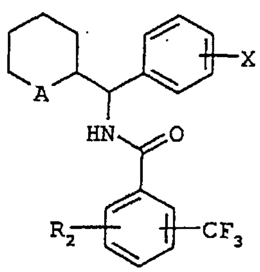

(I)

in which A represents
either a group of general formula N-$R_1$ in which $R_1$ represents either a hydrogen atom, or a linear or branched ($C_1$-$C_7$)alkyl group optionally substituted with one or more fluorine atoms, or a ($C_4$-$C_7$)cycloalkyl group, or a ($C_3$-$C_7$) cycloalkyl($C_1$-$C_3$)alkyl group, or a phenyl($C_1$-$C_3$)alkyl group optionally substituted with one or two hydroxyl or methoxy groups, or a ($C_2$-$C_4$)alkenyl group, or a ($C_2$-$C_4$)alkynyl group,
or a group of general formula $N^+(O^-)R_1$ in which $R_1$ is as defined above,
or alternatively a group of general formula $N^+(R')R_1$ in which R' represents a linear or branched ($C_1$-$C_7$)alkyl group and $R_1$ is as defined above,
X represents a hydrogen atom or one or more substituents chosen from halogen atoms and trifluoromethyl, linear or branched ($C_1$-$C_4$)alkyl and ($C_1$-$C_4$) alkoxy groups,
$R_2$ represents either a hydrogen atom, or one or more substituents chosen from halogen atoms and trifluoromethyl, ($C_1$-$C_4$) alkyl or ($C_1$-$C_4$) alkoxy groups, or amino groups of general formula $NR_3R_4$ in which $R_3$ and $R_4$ each represent, independently of each other, a hydrogen atom or a ($C_1$-$C_4$)-alkyl group, or form with the nitrogen atom carrying them a pyrrolidine, piperidine or morpholine ring, or a phenyl group optionally substituted with an atom or a group as defined for the symbol X above,
in the form of a free base or of an addition salt with an acid.

**2.** Compound according to Claim 1, **characterized in that** A represents a group of general formula N-$R_1$ in which $R_1$ represents either a hydrogen atom, or a linear or branched ($C_1$-$C_7$)alkyl group optionally substituted with one or more fluorine atoms, or a ($C_4$-$C_7$)cycloalkyl group, or a ($C_3$-$C_7$)cycloalkyl($C_1$-$C_3$)alkyl group, or a phenyl($C_1$-$C_3$)alkyl group optionally substituted with one or two hydroxyl or methoxy groups, or a ($C_2$-$C_4$)alkenyl group, or a ($C_2$-$C_4$) alkynyl group.

**3.** Compound according to Claim 1, **characterized in that** it has the configuration (1*S*,2*S*) and **in that** $R_2$ represents one or more halogen atoms or trifluoromethyl groups.

**4.** Compound according to Claim 1, **characterized in that** it has the configuration (1*R*,2*R*) and **in that** $R_2$ represents a halogen atom and an amino group of general formula $NR_3R_4$ as defined in Claim 1.

**5.** Compound according to Claim 1, **characterized in that** it is chosen from the following compounds:

- threo-2-chloro-*N*-[(1-ethylpiperidin-2-yl)phenylmethyl]-3-trifluoromethylbenzamide hydrochloride;
- 2-chloro-*N*-[(1*S*)-[(2*S*)-1-methylpiperidin-2-yl]phenylmethyl]-3-trifluoromethylbenzamide hydrochloride;
- 2-chloro-*N*-[(1*S*)-[(2*S*)-1-methylpiperidin-2-yl]phenylmethyl]-3-trifluoromethylbenzamide;
- threo-4-amino-3-chloro-*N*-[(1-methylpiperidin-2-yl)phenylmethyl]-5-trifluoromethylbenzamide hydrochloride;
- threo-4-amino-3-chloro-*N*-[(1-methylpiperidin-2-yl)phenylmethyl]-5-trifluoromethylbenzamide;
- 4-amino-3-chloro-*N*-[(1*R*)-[(2*R*)-1-methylpiperidin-2-yl]phenylmethyl]-5-trifluoromethylbenzamide hydrochloride;
- 4-amino-3-chloro-*N*-[(1*R*)-[(2*R*)-1-methylpiperidin-2-yl]phenylmethyl]-5-trifluoromethylbenzamide;
- threo-2-chloro-*N*-[phenyl(piperidin-2-yl)methyl]-3-trifluoromethylbenzamide hydrochloride;
- 2-chloro-*N*-[(*S*)-phenyl-[(2*S*)-piperidin-2-yl]methyl]-3-(trifluoromethyl)benzamide hydrochloride;
- 2-chloro-*N*-[(*S*)-phenyl-[(2*S*)-piperidin-2-yl]methyl]-3-(trifluoromethyl)benzamide;
- 2-chloro-N-[1-methyl-1-oxidopiperidin-2-yl](phenyl)methyl]-3-trifluoromethylbenzamide;
- (2*S*)-2[(1*S*)-[[2-chloro-3-(trifluoromethyl)benzoyl]-amino](phenyl)methyl]-1,1-dimethylpiperidinium iodide.

**6.** Compound according to Claim 1, **characterized in that** it is chosen from 2-chloro-*N*-[(*S*)-phenyl-[(2*S*)-piperidin-2-yl]methyl]-3-(trifluoromethyl)benzamide or its hydrochloride salt.

**7.** Medicament, **characterized in that** it consists of a compound according to one of Claims 1 to 6.

**8.** Pharmaceutical composition, **characterized in that** it contains a compound according to one of Claims 1 to 6, combined with an excipient.

**9.** Use of a compound according to Claim 1, for the preparation of a medicament intended for treating behavioural disorders linked to dementia, psychoses, schizophrenia (deficient form and productive form), for the treatment of various forms of anxiety, for the treatment of various forms of depression, including psychotic depression, for the treatment of disorders due to alcohol abuse or to alcohol withdrawal, sexual behaviour disorders, eating disorders, for the treatment of migraine, for the treatment of intense and/or chronic pain, for the treatment of Parkinson's disease, for the treatment of epilepsy and other epileptic syndromes in addition to another antiepileptic treatment.

## Patentansprüche

**1.** Verbindung in Form eines reinen optischen Isomers (1*R*, 2*R*) oder (1*S*, 2*S*) oder in Form eines threo-Diastereoisomers der allgemeinen Formel (I):

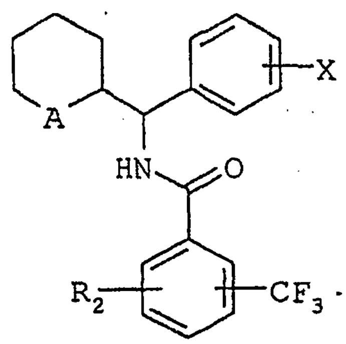

(I)

worin A
entweder für eine Gruppe der allgemeinen Formel N-R1 steht, worin R1 entweder für ein Wasserstoffatom oder eine lineare oder verzweigte $C_1$-$C_7$-Alkylgruppe steht, die eventuell durch eine oder mehrere Fluoratome oder eine $C_4$-$C_7$-Cycloalkylgruppe oder eine $C_3$-$C_7$-Cycloalkyl-$C_1$-$C_3$-alkylgruppe oder eine $C_1$-$C_3$-Alkylphenylgruppe substituiert ist, die eventuell durch eine oder zwei Hydroxy- oder Methoxygruppen oder eine $C_2$-$C_4$-Alkenylgruppe oder eine $C_2$-$C_4$-Alkinylgruppe substituiert ist,
oder eine Gruppe der allgemeinen Formel $N^+(O^-)R_1$, worin $R_1$ wie oben definiert ist,
oder auch eine Gruppe der allgemeinen Formel $N^+(R')R_1$, worin R' für eine lineare oder verzweigte $C_1$-$C_7$-Alkylgruppe steht und $R_1$ wie oben definiert ist,
X für ein Wasserstoffatom oder einen oder mehrere Substituenten steht, ausgewählt aus den Halogenatomen und Trifluormethyl-, linearen oder verzweigten $C_1$-$C_4$-Alkyl- und $C_1$-$C_4$-Alkoxygruppen,
$R_2$ entweder für ein Wasserstoffatom oder einen oder mehrere Substituenten steht, ausgewählt aus Halogenatomen und Trifluormethyl-, $C_1$-$C_4$-Alkyl-, $C_1$-$C_4$-Alkoxy-, Aminogruppen der allgemeinen Formel $NR_3R_4$, worin $R_3$ und $R_4$ jeweils unabhängig voneinander für ein Wasserstoffatom oder eine $C_1$-$C_4$-Alkylgruppe stehen, oder mit dem Stickstoffatom, das sie trägt, einen Pyrrolidin-, Piperidin- oder Morpholinring bilden, oder eine Phenylgruppe, die eventuell durch ein Atom oder eine Gruppe substituiert ist, wie oben für das Symbol X definiert,
im Zustand einer freien Base oder eines Säureadditionssalzes.

2. Verbindung nach Anspruch 1, **dadurch gekennzeichnet, dass** A für eine Gruppe der allgemeinen Formel N-$R_1$ steht, worin $R_1$ entweder für ein Wasserstoffatom oder eine lineare oder verzweigte $C_1$-$C_7$-Alkylgruppe steht, die eventuell durch ein oder mehrere Fluoratome substituiert ist, oder eine $C_4$-$C_7$-Cycloalkylgruppe oder eine $C_3$-$C_7$-Cycloalkyl-$C_1$-$C_3$-alkylgruppe oder eine $C_1$-$C_3$-Alkylphenylgruppe, die eventuell durch eine oder zwei Hydroxy- oder Methoxygruppen oder eine $C_2$-$C_4$-Alkenylgruppe oder eine $C_2$-$C_4$-Alkinylgruppe substituiert ist.

3. Verbindung nach Anspruch 1, **dadurch gekennzeichnet, dass** sie die Konfiguration (1S, 2S) aufweist und **dadurch**, dass $R_2$ für ein oder mehrere Halogenatome oder Trifluormethylgruppen steht.

4. Verbindung nach Anspruch 1, **dadurch gekennzeichnet, dass** sie die Konfiguration (1R, 2R) aufweist und **dadurch**, dass $R_2$ für ein Halogenatom und eine Aminogruppe mit der allgemeinen Formel $NR_3R_4$ steht, wie in Anspruch 1 definiert.

5. Verbindung nach Anspruch 1, **dadurch gekennzeichnet, dass** sie aus den folgenden Verbindungen ausgewählt ist:

- threo-2-Chlor-N-[(1-ethylpiperidin-2-yl)phenylmethyl]-3-trifluormethylbenzamid-chlorhydrat;
- 2-Chlor-N-[(1S)-[(2S)-1-methylpiperidin-2-yl]phenylmethyl)-3-trifluormethylbenzamid-chlorhydrat;
- 2-Chlor-N-[(1S)-[(2S)-1-methylpiperidin-2-yl]phenylmethyl]-3-trifluormethylbenzamid;
- threo-4-Amino-3-chlor-N-[(1-methylpiperidin-2-yl)phenylmethyl]-5-trifluormethylbenzamid-chlorhydrat;
- threo-4-Amino-3-chlor-N-[(1-methylpiperidin-2-yl)phenylmethyl]-5-trifluormethylbenzamid;
- 4-Amino-3-chlor-N-[(1R)-(2R)-1-methylpiperidin-2-yl]phenylmethyl]-5-trifluormethylbenzamid-chlorhydrat;
- 4-Amino-3-chlor-N-[(1R)-[(2R)-1-methylpiperidin-2-yl]phenylmethyl]-5-trifluormethylbenzamid;
- threo-2-Chlor-N-[phenyl(piperidin-2-yl)methyl]-3-trifluormethylbenzamid-chlorhydrat;
- 2-Chlor-N-[(S)-phenyl-[(2S)-piperidin-2-yl]methyl]-3-(trifluormethyl)benzamid-chlorhydrat;
- 2-Chlor-N-[(S)-phenyl-[(2S)-piperidin-2-yl]methyl]-3-(trifluormethyl)benzamid;
- 2-Chlor-N-[[1-methyl-1-oxido-piperidin-2-yl](phenyl)methyl]-3-trifluormethylbenzamid;

- (2S)-2[(1S)-[[2-Chlor-3-(trifluormethyl)benzoyl]-amino](phenyl)methyl]-1,1-dimethylpiperidiniumiodid.

6. Verbindung nach Anspruch 1, **dadurch gekennzeichnet, dass** sie ausgewählt ist aus 2-Chlor-N-[(S)-phenyl-[(2S)-piperidin-2-yl]methyl]-3-(trifluormethyl)benzamid oder dessen Chlorhydratsalz.

7. Medikament, **dadurch gekennzeichnet, dass** es aus einer Verbindung nach einem der Ansprüche 1 bis 6 besteht.

8. Pharmazeutische Zusammensetzung, **dadurch gekennzeichnet, dass** sie eine Verbindung nach einem der Ansprüche 1 bis 6 enthält, die an einen Hilfsstoff gebunden ist.

9. Verwendung einer Verbindung nach Anspruch 1 zur Herstellung eines Medikaments, das zur Behandlung von Verhaltensstörungen, die mit Demenz, Psychosen, Schizophrenie (defizitäre und produktive Form) verbunden sind, zur Behandlung diverser Angstformen, zur Behandlung verschiedener Formen der Depression, einschließlich der psychotischen Depression, zur Behandlung von Störungen, die auf Alkoholmissbrauch oder Alkoholentzug zurückzuführen sind, Störungen des Sexualverhaltens, Störungen der Nahrungsaufnahme, zur Behandlung von Migräne, zur Behandlung von starken und/oder chronischen Schmerzen, zur Behandlung der Parkinsonkrankheit, zur Behandlung von Epilepsien und anderen epileptischen Syndromen als Ergänzung einer anderen antiepileptischen Behandlung, bestimmt ist.